# EUROPÄISCHE PATENTSCHRIFT

(11) **EP 2 606 934 B1**
(45) Veröffentlichungstag und Bekanntmachung des Hinweises auf die Patenterteilung: **27.02.2019**
(21) Anmeldenummer: 12197343.2
(22) Anmeldetag: 14.12.2012
(51) Int. Cl.: A61Q 5/06, A61K 8/36, A61K 8/40, A61Q 5/12, A61K 8/04

(54) **Verwendung von Octanohydroxamsäure in Haarstylingformulierungen**
Hair styling formulation for the creation of a flexible hold by means of octanohydroxamic acid
Formule de coiffage de cheveux destinée à produire une tenue flexible en utilisant de l'acide octanohydroxamique

(30) Priorität: 19.12.2011 DE 102011088926
(43) Veröffentlichungstag der Anmeldung: 26.06.2013
(73) Patentinhaber: Beiersdorf AG, 20253 Hamburg (DE)
(72) Erfinder: Sors, Nathalie, 21643 Beckdorf (DE); Wächter, Anna Christina, 20251 Hamburg (DE); Köhler, Manuela, 22147 Hamburg (DE); Göksel, Hülya, 50733 Köln (DE)

(56) Entgegenhaltungen:
- EP-A1- 0 498 636
- EP-A2- 0 313 347
- EP-A2- 2 308 837
- WO-A1-2009/070736
- WO-A2-2006/029818
- US-A1- 2006 084 586
- DATABASE GNPD [Online] MINTEL; 1. November 2009 (2009-11-01), Kroger: "Mirra Daily Volumizing Conditioner", XP002724337, Database accession no. 1211363
- DATABASE GNPD [Online] MINTEL; 1. Juni 2009 (2009-06-01), Philip B: "Katira Hair Masque", XP002724338, Database accession no. 1126285
- DATABASE GNPD [Online] MINTEL; 1. Dezember 2010 (2010-12-01), Evelyn Products: "FroHim Mointain Mist Cleansing Foam", XP002724339, Database accession no. 1453090
- DATABASE GNPD [Online] MINTEL; 1. Dezember 2010 (2010-12-01), Evelyn Products: "FroHer Aloe & Honey Haze Comb-out Hair Mist", XP002724340, Database accession no. 1452845

## Beschreibung

Die vorliegende Erfindung betrifft Haarstylingmittel mit einem Gehalt an einer oder mehreren physiologisch unbedenklichen Hydroxamsäuren, insbesondere Octanohydroxamsäure.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem sogenannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Natürliches Haar hängt meist schlaff und ohne Volumen vom Kopf herab. Ein Ziel der Haarpflege ist es daher, dem Haar Fülle und Frisur zu geben. Zur temporären Verformung des Haares und Gestaltung (engl. Styling) vielseitiger Frisuren verwendet man Stylingmittel (Haarfestiger), meist in Form von Schaumfestigern, Haargelen oder Haarsprays. Schaumfestiger (auch Haarschaumfestiger genannt) werden in der Regel zur Frisurgestaltung im feuchten Haar verteilt, Haarsprays zur Fixierung auf die (trockene) fertig gestylte Frisur gesprüht. Neben den wegen ihrer leichten und angenehmen Anwendbarkeit zunehmend beliebten Schaumhaarfestigern und flüssigen Haarfestigern werden auch Haarfestigergele (Haargele) angeboten.

Die beiden Grundbausteine von Haarfestigern sind die Filmbildner (d.h. fixierende Polymere) und das Lösungsmittel. Als Filmbildner werden meist nichtionische, anionische, kationische oder amphothere Polymere eingesetzt. Filmbildner bleiben nach dem Verdunsten des Lösungsmittels (meist Wasser und/oder Ethanol) als Film auf dem Haar und fixieren dessen Form. An den auf dem Haar abgelagerten Film werden eine Vielzahl an Anforderungen gestellt: Er soll klar, feuchtigkeitsunempfindlich, festigend, stabil und nicht klebrig sein sowie sich leicht durch handelsübliche Haarshampoos auswaschen lassen. Neben den Filmbildnern enthalten Stylingzubereitungen Verdickungsmittel, die dem Produkt die gewünschte Konsistenz geben.

Herkömmliche Haarfestiger, insbesondere Haargele Haarsprays und Schaumfestiger, haben eine Reihe von Nachteilen. So weisen die in ihnen enthaltenden Filmbildner zwar meist eine hohe Festigungsleistung auf, doch sind die Filme auf dem Haar in der Regel spröde und hinterlassen insbesondere beim Auskämmen Rückstände auf Haar und Kleidung. Diese Rückstände, auch Filmplaken genannt, verleihen dem Anwender ein ungepflegtes Aussehen. Es entsteht der Eindruck, der Anwender leide unter Schuppen-Kopfhaut. Dieses Phänomen tritt insbesondere dann (und insbesondere bei Gelen) auf, wenn kationische Verdickungsmittel eingesetzt werden. Werden anionische Verdickungsmittel eingesetzt, können die Zubereitungen darüber hinaus keine kationischen Pflegestoffe (beispielsweise polyquaternierte Verbindungen) enthalten, was aus Gründen der Haarpflege durchaus wünschenswert wäre.

Aus diesen Gründen werden seit geraumer Zeit teils Haarpflegekosmetika verwendet, welche dazu bestimmt sind, nach Einwirken aus dem Haar wieder ausgespült zu werden, teils solche, welche auf dem Haar verbleiben sollen. Letztere können so formuliert werden, daß sie nicht nur der Pflege des einzelnen Haars dienen, sondern auch das Aussehen der Haartracht insgesamt verbessern, beispielsweise dadurch, daß sie dem Haar mehr Fülle verleihen, die Haartracht über einen längeren Zeitraum fixieren oder seine Frisierbarkeit verbessern.

Die Eigenschaft der Fülle wird einer Frisur beispielsweise zugeschrieben, wenn das Haar nach der Behandlung nicht flach auf der Kopfhaut aufliegt und gut frisierbar ist.

Die Eigenschaft des Volumen wird einer Frisur beispielsweise zugeschrieben, wenn das Haar nach der Behandlung Fülle und Sprungkraft aufweist.

Die Eigenschaft des Body's wird einer Frisur beispielsweise zugeschrieben, wenn das Haarvolumen selbst unter äußeren, störenden Einflüssen groß bleibt.
Unter Curl Retention versteht man das Aushängeverhalten von Haarlocken. Der erzeugte Index-Wert ist hierbei umso besser, je weiniger sich auf Lockenwickler gedrehte und mit Stylingprodukten fixierte Haarsträhnen unter dem Einfluss der Schwerkraft und der Umgebungsfeuchte nach unten hin verlängern.
Durch quaternäre Ammoniumverbindungen beispielsweise läßt sich die Kämmbarkeit der Haare entscheidend verbessern. Solche Verbindungen ziehen auf das Haar auf und sind oft noch nach mehreren Haarwäschen auf dem Haar nachweisbar.

Haarpflegeprodukte enthaltend Caprylhydroxamsäure waren auf dem Markt, z.B. "Mirra Daily" Conditioner der Firma Kroger (Mintel GNPD Record ID 1211363) und "Katira Hair Mask" der Firma Philip B (Mintel GNPD Record ID 1126285).

Der Stande der Technik ließ es aber an Wirkstoffen und Zubereitungen mangeln, welche dem geschädigten Haar in befriedigender Weise Pflege zukommen ließen. Auch erwiesen sich Zubereitungen, die der Haartracht Fülle geben sollten, oft als unzureichend, zumindest waren sie ungeeignet, als Haarpflegezubereitungen eingesetzt zu werden. Die Haartracht fixierende Zubereitungen des Standes der Technik enthalten beispielsweise in der Regel viskose Bestandteile, welche Gefahr laufen, ein Gefühl der Klebrigkeit zu erwecken, welches oft durch geschickte Formulierung kompensiert werden muß.
Aufgabe war daher, den Nachteilen des Standes der Technik Abhilfe zu schaffen.
Es war daher die Aufgabe der vorliegenden Erfindung die Mängel des Standes der Technik zu mindern, im Idealfalle zu beseitigen.
Gele bestehen üblicherweise aus einem verdicktem wässrigen bzw. wässrig-alkoholischen Lösung von filmbildenden natürlichen oder synthetischen Polymeren. Diese Polymere können aus der Gruppe der nichtionischen, kationischen, amphoteren oder anionischen Polymeren ausgewählt werden. Der Polymerfilm von Gelen härtet meist vollständig aus, weshalb eine sehr starke Festigung erzielt werden kann. Diese Filme sind jedoch sehr spröde und lassen sich leicht zerstören.
Handelsübliche Wachse erzielen eine flexible und nicht spröde, aber nicht starke Festigung. Sie bestehen aus einer Mischung von Wachsen, Ölen, Fetten und Emulgatoren.

Die üblicherweise eingesetzten Polymere, wie oben beschrieben, können in Haar-Wachsen nur gering festigende Filme ausbilden, da die Festigung von Fetten, Ölen und Wachsen negativ beeinflusst wird.

Es hat sich für den Fachmann überraschend und nicht vorhersehbar herausgestellt, daß Haarkosmetische Zubereitungen mit einem Gehalt 0,01 bis 10 Gew.-%, insbesondere 0,05 bis 3,0 Gew.-%, an physiologisch verträglicher Octanohydroxamsäure, bezogen auf das Gesamtgewicht der Zusammensetzung und
- einem oder mehreren kationischen Polymeren, welches oder welche gewählt werden aus der Gruppe Vinylpyrrolidon/Vinylimidazolium-methochlorid-Copolymere, quaternisierte Vinylpyrrolidon/Dialkylaminoalkylmethacrylat-Copolymere, kationische Cellulose-Derivate, wie z.B. Hydroxyethylcellulose/Dimethyl-alkylammoniumchlorid-Copolymere sowie Terpolymere aus Vinylcaprolactam/-Vinylpyrrolidon mit Dimethylaminoethylmethacrylat bzw. Vinylimmidazoliniummetho-chlorid und Acrylamidocopolymere, Hydroxyethyl Cellulose Dimethyl Diallylammonium Chloride Copolymer (Polyquaternium-4), Vinylpyrrolidon/ Vinylimidazol/Methacrylamid/-quaternisiertes Vinylimidazol (Polyquaternium-68), Vinylpyrrolidone/ quaternisiertes Dimethylaminoethylmethacrylate Copolymer (Polyquaternium-11), vorzugsweise Polyquaternium-4, Polyquaternium-11 und Polyquaternium-68 und
- einem oder mehreren nichtionischen Polymeren, welches oder welche gewählt werden aus der Gruppe Homopolymere des Vinylpyrrolidons, Homopolymere des N-Vinylformamids, Copolymierisate aus Vinylpyrrolidon und Vinylacetat, Terpolymere aus Vinylpyrrolidon, Vinylacetat und Propionat, Polyvinylcaprolactam, Polyvinylamide und deren Salze sowie Copolymere aus Vinylpyrrolidon und Dimethylaminoethylmethacrylat und Terpolymere aus Vinylcaprolactam, Vinylpyrrolidon und Dimethylaminomethacrylat, vorzugsweise Copolymerisate aus Vinylpyrrolidon und Vinylacetat sowie Homopolymerisate des Vinylpyrrolidons
den Nachteilen des Standes der Technik abhelfen.

Der Begriff "Verbesserung der Curl-Retention-Eigenschaften menschlicher Haare" bedeutet, daß eine entsprechende Rezeptur, die sich durch einen Gehalt an einer oder mehreren physiologisch verträglichen Hydroxamsäuren, insbesondere Octanohydroxamsäure, aufweist, bestimmungsgemäß angewandt, zu besserer Curl-Retention-Eigenschaften des menschlichen Haares führt, als nach Anwendung einer entsprechende Rezeptur ohne Hydroxamsäure.

Dabei wird verglichen eine Rezeptur, die sich durch einen Gehalt an einer oder mehreren physiologisch verträglichen Hydroxamsäuren, insbesondere Octanohydroxamsäure, aufweist, mit einer solchen, bei der der Gewichtsanteil der Hydroxamsäure durch einen entsprechenden Gewichtsanteil an Wasser oder der Hauptölkomponente der Zubereitung ersetzt worden ist.

Der Begriff "Verbesserung der Fixierbarkeit menschlicher Haare oder deren Frisierbarkeit" bedeutet, daß eine entsprechende Rezeptur, die sich durch einen Gehalt an einer oder mehreren physiologisch verträglichen Hydroxamsäuren, insbesondere Octanohydroxamsäure, aufweist, bestimmungsgemäß angewandt die Frisur leichter frisiert bzw. besser fixiert, als eine entsprechende Rezeptur ohne Hydroxamsäure.

Dabei wird verglichen eine Rezeptur, die sich durch einen Gehalt an einer oder mehreren physiologisch verträglichen Hydroxamsäuren, insbesondere Octanohydroxamsäure, aufweist, mit einer solchen, bei der der Gewichtsanteil der Hydroxamsäure durch einen entsprechenden Gewichtsanteil an Wasser oder der Hauptölkomponente der Zubereitung ersetzt worden ist.

Der Begriff "Erhöhung der Haarfülle" bedeutet, daß eine entsprechende Rezeptur, die sich durch einen Gehalt an Octanohydroxamsäure aufweist, bestimmungsgemäß angewandt zu größerer Haarfülle führt, als eine entsprechende Rezeptur ohne Octanohydroxamsäure.

Dabei wird verglichen eine Rezeptur, die sich durch einen Gehalt an einer oder mehreren physiologisch verträglichen Hydroxamsäuren, insbesondere Octanohydroxamsäure, aufweist, mit einer solchen, bei der der Gewichtsanteil der Hydroxamsäure durch einen entsprechenden Gewichtsanteil an Wasser oder der Hauptölkomponente der Zubereitung ersetzt worden ist.

Der Begriff "Erhöhung des Haarvolumens" bedeutet, daß eine entsprechende Rezeptur, die sich durch einen Gehalt an einer oder mehreren physiologisch verträglichen Hydroxamsäuren, insbesondere Octanohydroxamsäure, aufweist, bestimmungsgemäß angewandt zu größerem Haarvolumen führt, als eine entsprechende Rezeptur ohne Hydroxamsäure.

Dabei wird verglichen eine Rezeptur, die sich durch einen Gehalt an einer oder mehreren physiologisch verträglichen Hydroxamsäuren, insbesondere Octanohydroxamsäure, aufweist, mit einer solchen, bei der der Gewichtsanteil der Hydroxamsäure durch einen entsprechenden Gewichtsanteil an Wasser oder der Hauptölkomponente der Zubereitung ersetzt worden ist.

Der Begriff "Verbesserung des Haarbodys" bedeutet, daß eine entsprechende Rezeptur, die sich durch einen Gehalt an einer oder mehreren physiologisch verträglichen Hydroxamsäuren, insbesondere Octanohydroxamsäure, aufweist, bestimmungsgemäß angewandt zu besserem Haarbody führt, als eine entsprechende Rezeptur ohne Hydroxamsäure.

Dabei wird verglichen eine Rezeptur, die sich durch einen Gehalt an einer oder mehreren physiologisch verträglichen Hydroxamsäuren, insbesondere Octanohydroxamsäure, aufweist, mit einer solchen, bei der der Gewichtsanteil der Hydroxamsäure durch einen entsprechenden Gewichtsanteil an Wasser oder der Hauptölkomponente der Zubereitung ersetzt worden ist

Der Begriff "Erhöhung der Haarelastizität" bedeutet, daß eine entsprechende Rezeptur, die sich durch einen Gehalt an einer oder mehreren physiologisch verträglichen Hydroxamsäuren, insbesondere Octanohydroxamsäure, aufweist, bestimmungsgemäß angewandt zu höherer Haarelastizität führt, als eine entsprechende Rezeptur ohne Hydroxamsäure.

Dabei wird verglichen eine Rezeptur, die sich durch einen Gehalt an einer oder mehreren physiologisch verträglichen Hydroxamsäuren, insbesondere Octanohydroxamsäure, aufweist, mit einer solchen, bei der der Gewichtsanteil der Hydroxamsäure durch einen entsprechenden Gewichtsanteil an Wasser oder der Hauptölkomponente der Zubereitung ersetzt worden ist

Der Begriff "Verbesserung der Curl Retention" bedeutet, daß eine entsprechende Rezeptur, die sich durch einen Gehalt an einer oder mehreren physiologisch verträglichen Hydroxamsäuren, insbesondere Octanohydroxamsäure, aufweist, bestimmungsgemäß angewandt zu höherer Haarelastizität führt, als eine entsprechende Rezeptur ohne Hydroxamsäure. → doppelt, siehe ersten Punkt, außerdem inhaltlich fraglich, Haarelastizität und Curl Retention ist nicht das Gleiche.

Auch hier fehlt der Teil mit der Halte- und Festigungsperformance.

Dabei wird verglichen eine Rezeptur, die sich durch einen Gehalt an einer oder mehreren physiologisch verträglichen Hydroxamsäuren, insbesondere Octanohydroxamsäure, aufweist, mit einer solchen, bei der der Gewichtsanteil der Hydroxamsäure durch einen entsprechenden Gewichtsanteil an Wasser oder der Hauptölkomponente der Zubereitung ersetzt worden ist

Octanohydroxamsäure, auch Caprylohydroxamsäure genannt, ist durch folgende Struktur gekennzeichnet.

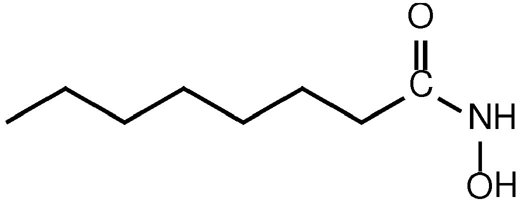

Sie ist ein bekannter Wirkstoff zur Konservierung kosmetischer Zubereitungen (z.B. WO 2009/070736 A1)

Die Stylingmittel und Zubereitungen, die erfindungsgemäße Wirkstoffkombinationen enthalten, sind topische Zubereitungen. Diese können wie üblich zusammengesetzt sein und zur Behandlung und der Pflege der Kopfhaut und/oder der Haare dienen. Zur Anwendung werden die erfindungsgemäßen Zubereitungen in der für Kosmetika und Stylingmittel üblichen Weise auf die Kopfhaut und die Haare in ausreichender Menge aufgebracht.

Vorteilhaft können Zubereitungen im Sinne der vorliegenden Erfindung als Haarsprays oder Flüssigfestiger oder Lösungen vorliegen.

Die Mittel gemäß der Erfindung können beispielsweise als aus Aerosolbehältern, Quetschflaschen oder durch eine Pump-Sprüh-Vorrichtung versprühbare Präparate vorliegen, jedoch auch in Form eines aus normalen Flaschen und Behältern auftragbaren Mittels.

Als Treibmittel für aus Aerosolbehältern versprühbare kosmetische oder dermatologische Zubereitungen im Sinne der vorliegenden Erfindung, wie z.B. Haarsprays, sind die üblichen bekannten leichtflüchtigen, verflüssigten Treibmittel, beispielsweise Dimethylether, Kohlenwasserstoffe (Propan, Butan, Isobutan) geeignet, die allein oder in Mischung miteinander eingesetzt werden können. Auch Druckluft, Stickstoff, Stickstoffdioxid oder Kohlendioxid oder Gemische aus diesen Substanzen sind vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Vorteilhaft enthalten erfindungsgemäße Zubereitungen neben einem wirksamen Gehalt an erfindungsgemäßen Wirkstoffkombinationen ferner übliche Wirk-, Inhalts-, Zusatz- und/oder Hilfsstoffe.

Erfindungsgemäße kosmetische Zubereitungen zur Festigung und zum Styling der Haare enthalten üblicherweise Filmbildner, wie sie normalerweise in solchen Zubereitungen verwendet werden, wobei die Gesamtmenge der Filmbildnersubstanzen z. B. zwischen 0,5 und 20,0 Gew.-% beträgt, bezogen auf das Gesamtgewicht der Zubereitungen.

Erfindungsgemäß können als günstige Filmbildner alle für kosmetische und/oder dermatologische Anwendungen geeigneten oder gebräuchlichen Filmbildner verwendet werden.

Vorteilhaft werden der oder die Filmbildner gewählt aus der Gruppe der alkohollöslichen oder alkoholdispergierbaren Polyurethane, Polyharnstoffe, Silikonharze und/oder Polyester sowie der nichtionischen, anionischen, amphoteren und/oder kationischen Polymere.

Vorteilhafte anionische Polymere sind beispielsweise Vinylacetat/Crotonsäure-, Vinylacetat/Acrylat- und/oder Vinylacetat/Vinylneodecanoat/Crotonsäure-Copolymere, Natriumacrylat/Vinylalkohol-Copolymere, Natriumpolystyrolsulfonat, Ethylacrylat/N-tert.-Butylacrylamid/Acrylsäure-Copolymere, Vinylpyrrolidon/Vinylacetat/Itaconsäure-Copolymere, Acrylsäure/Acrylamid-Copolymere und/oder deren Natriumsalze, Homo- und/oder Copolymere von Acrylsäure und/oder Methacrylsäure und/oder deren Salze sowie Acrylat/Hydroxyacrylat-, Octylacrylamid/Acrylat- bzw. Methacrylsäurester und/oder Butylacrylat/N-Vinylpyrrolidon-Copolymere.

Weitere bevorzugte anionische Polymere sind Methylvinylether/Maleinsäure-Copolymere, die durch Hydrolyse von Vinylether/Maleinsäureanhydrid-Copolymeren entstehen. Diese Polymere können auch teilverestert sein (Ethyl, Isopropyl- bzw. Butylester).

Vorteilhafte amphotere Polymere, die in erfindungsgemäßen Zubereitungen alleine oder im Gemisch, vorzugsweise auch mit anionischen und/oder nichtionischen Polymeren enthalten sein können, sind Copolymerisate aus N-Octylacrylamid, (Meth)Acrylsäure und tert.-Butylaminoethylmethacrylat vom Typ "Amphomer", Copolymerisate aus Methacryloylethylbetain und Alkylmethacrylaten vom Typ "Yukaformer", Copolymerisate aus Carboxylgruppen oder Sulfongruppen enthaltenden Monomeren, z.B. (Meth)Acrylsäure und Itaconsäure, mit basischen, insbesondere Aminogruppen enthaltenden Monomeren wie z.B. Mono- bzw. Dialkylaminoalkyl(meth)acrylaten und/oder Mono- bzw. Dialkylaminoalkyl(meth)acrylamiden, Copolymere aus N-Octylacrylamid, Methylmethacrylat, Hydroxypropylmethacrylat, N-tert.-Butylaminoethylmethacrylat und Acrylsäure, wobei diese Aufstellung selbstverständlich nicht limitierend sein soll.

Es ist gegebenenfalls vorteilhaft, die anionischen und amphoteren Polymere zur Verbesserung ihrer Wasserlöslichkeit bzw. Treibmittelverträglichkeit mit geeigneten Basen zu neutralisieren. Hierzu können beispielsweise Alkali- bzw. Erdalkalibasen, Ammoniak und/oder verschiedene Amine, wie z.B. Triethanolamin, Triisopropanolamin, Aminomethyl-propanol und/oder Aminomethylpropandiol, eingesetzt werden. Die Neutralisation kann je nach Anwendungszweck teilweise oder vollständig erfolgen.

Vorteilhafte Verkörperungen der vorligenden Erfindungen sind daher auch haarkosmetische Zubereitungen mit einem Gehalt an einer oder mehreren physiologisch verträglichen Hydroxamsäuren, insbesondere Octanohydroxamsäure und einem oder mehreren kationischen Polymeren.

Es ist auch vorteilhaft, Filmbildner auf natürlicher Basis, z.B. Chitosan und dessen Derivate, einzusetzen, insbesondere im Gemisch mit synthetischen Polymeren.

Kationische Tenside werden zumeist ausgewählt aus der Gruppe der quaternären Ammoniumverbindungen, insbesondere Benzyltrialkylammoniumsalze oder Alkyltrimethylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Hydroxyethyl Cetyldimonium Phosphate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide.

Vorteilhafte Verkörperungen der vorligenden Erfindungen sind daher auch haarkosmetische Zubereitungen mit einem Gehalt an einer oder mehreren physiologisch verträglichen Hydroxamsäuren, insbesondere Octanohydroxamsäure und einem oder mehreren kationischen Tensiden.

Die erfindungsgemäßen Zubereitungen können kosmetische Hilfsstoffe enthalten, wie sie üblicherweise in solchen Zubereitungen verwendet werden, z.B. Parfüme, Substanzen zum Verhindern des Schäumens, Farbstoffe, Pigmente, die eine färbende Wirkung haben, oberflächenaktive Substanzen, Lösungsvermittler, weichmachende, anfeuchtende und/oder feuchhaltende Substanzen, rückfettende Agentien, Alkohole, Polyole und deren toxikologisch verträglichen Ether und Ester, verzweigte und/oder unverzweigte Kohlenwasserstoffe, weitere Antioxidantien, Stabilisatoren, pH-Wert-Regulatoren, Konsistenzgeber, Bakterizide, Desodorantien, antimikrobielle Stoffe, Antistatika, UV-Absorber, Polymere, Elektrolyte, organische Lösungsmittel, Silikonderivate, Pflanzenextrakte, Vitamine und/oder andere Wirkstoffe oder andere übliche Bestandteile einer kosmetischen oder dermatologischen Formulierung.

Die Gesamtmenge der Hilfsstoffe beträgt beispielsweise 0,001 bis 15 Gew.-%, vorzugsweise 0,01 bis 10 Gew.-%, jeweils bezogen auf das Gesamtgewicht der Zubereitung.

Die erfindungsgemäßen Zubereitungen auf Basis von Alkohol enthalten vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin.

Der Anteil der Alkohole in den Zubereitungen beträgt beispielsweise 10 bis 99 Gew.-%.

Die Menge der Alkohole in Aerosol-Zubereitungen beträgt beispielsweise 20 bis 60 Gew.-%, vorzugsweise 30 bis 60 Gew.-%, jeweils bezogen auf das Gesamtgewicht der Zubereitung.

Die Menge der Alkohole in Non-Aerosol-Zubereitungen beträgt beispielsweise 30 bis 99 Gew.-%, vorzugsweise 60 bis 99 Gew.-%, jeweils bezogen auf das Gesamtgewicht der Zubereitung.

Die erfindungsgemäßen Zubereitungen können Wasser z.B. in den durch die verwendeten Rohstoffe eingebrachten Mengen enthalten, beispielsweise oder z.B. auch in Mengen von bis zu 30 Gew.-%, bezogen auf das Gesamtgewicht der Zubereitungen.

Erfindungsgemäß können als weitere Antioxidantien alle für kosmetische und/oder dermatologische Anwendungen geeigneten oder gebräuchlichen Antioxidantien verwendet werden.

Die Gesamtmenge der Antioxidantien beträgt beispielsweise 0,001 bis 2 Gew.-%, vorzugsweise 0,01 bis 1 Gew.-%, jeweils bezogen auf das Gesamtgewicht der Zubereitung.

Vorteilhaft können erfindungsgemäße Zubereitungen außerdem Substanzen enthalten, die UV-Strahlung im UV-B-Bereich absorbieren, wobei die Gesamtmenge der Filtersubstanzen z.B. 0,001 Gew.-% bis 30 Gew.-%, vorzugsweise 0,05 bis 10 Gew.-%, insbesondere 0,1 bis 1,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut, insbesondere die Kopfhaut dienen.

Enthalten die erfindungsgemäßen Emulsionen UV-B-Filtersubstanzen, können diese vorteilhaft wasserlöslich sein. Vorteilhafte wasserlösliche UV-B-Filter sind z.B.:
- Salze der 2-Phenylbenzimidazol-5-sulfonsäure wie ihr Natrium-, Kalium- oder ihr Triethanolammonium-Salz, sowie die 2-Phenylbenzimidazol-5-sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Es kann auch von Vorteil sein, erfindungsgemäße Zubereitungen mit UV-A-Filtern zu versetzen, die bisher üblicherweise in kosmetischen Zubereitungen enthalten sind. Es können die für die UV-B-Kombination verwendeten Mengen eingesetzt werden.

In den erfindungsgemäßen Stylingformulierungen können als Filmbildner bevorzugt nichtionische oder amphotere Polymere eingesetzt werden.

Nichtionische Polymere, wie z.B. PVP/VA Copolymere, die z.B. von der Gesellschaft BASF unter dem Handelsnamen Luviskol VA 37E bzw. von der Gesellschaft International Speciality Products (ISP) unter der Bezeichnung PVP/VA E 335 verfügbar sind, werden dabei z.B. in Konzentrationen von 2 - 8 Gew.-% des Gesamtgwichts der Zubereitung (bezogen auf den Aktivgehalt des Polymers) eingesetzt.

Amphotere Polymere vom Typ Octylacrylamide/Acrylates/Butylaminoethyl Methacrylate Copolymer sind z.B. von der Gesellschaft National Starch unter den Handelsbezeichnungen Amphomer 28-4910 und LV-71 verfügbar und werden, z.B. in Konzentrationen zwischen 1 - 6 Gew.-% des Gesamtgewichts der Zubereitung eingesetzt.

In den erfindungsgemäßen Stylingformulierungen auf alkoholischer Basis können als Pflegestoffe bevorzugt cyclische Polydimethylsiloxane (Cyclomethicone) in Konzentrationen von z.B. 0.1 - 1.0 Gew.-% der Gesamtformulierung oder Silikontenside (Polyethermodifizierte Siloxane) vom Typ Dimethicone Copolyol z.B. in Konz. 0.01 - 1.0 Gew.-% des Gesamtgewichts der Zubereitung bevorzugt zum Einsatz kommen.

Cyclomethicone werden u.a. unter der Handelsbezeichnungen Abil K4 von der Gesellschaft Goldschmidt oder z.B. DC 244, DC 245 oder DC 345 von der Gesellschaft Dow Corning angeboten.

Dimethicone Copolyole werden z.B. unter der Handelsbezeichnung DC 193 von der Gesellschaft Dow Corning bzw. Belsil DM 6031 von der Gesellschaft Wacker angeboten.

Es kann gegebenenfalls erfindungsgemäß vorteilhaft sein, den erfindungsgemäß verwendeten kosmetischen oder dermatologischen Zubereitungen Komplexbildner zuzufügen.

Komplexbildner sind an sich bekannte Hilfsstoffe der Kosmetologie bzw. der medizinischen Galenik. Durch die Komplexierung von störenden Metallen wie Mn, Fe, Cu und anderer können beispielsweise unerwünschte chemische Reaktionen in kosmetischen oder dermatologischen Zubereitungen verhindert werden.

Komplexbildner, insbesondere Chelatoren, bilden mit Metallatomen Komplexe, welche bei Vorliegen eines oder mehrerer mehrbasiger Komplexbildner, also Chelatoren, Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom od. -Ion zu Ringen geschlossen. Die Zahl der gebundenen Liganden hängt von der Koordinationszahl des zentralen Metalls ab. Voraussetzung für die Chelatbildung ist, daß die mit dem Metall reagierende Verbindung zwei oder mehr Atomgruppierungen enthält, die als Elektronendonatoren wirken.

Der oder die Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen).

Der oder die Komplexbildner sind erfindungsgemäß vorteilhaft in kosmetischen oder dermatologischen Zubereitungen bevorzugt zu 0,01 Gew.-% bis 10 Gew.-%, bevorzugt zu 0,05 Gew.-% bis 5 Gew.-%, insbesondere bevorzugt zu 0,1 - 2,0 Gew.-%, bezogen auf das Gesamtgewicht der Zubereitungen, enthalten.

Die Herstellung der erfindungsgemäßen Zubereitungen kann in der üblichen Weise durch Mischen der einzelnen Bestandteile erfolgen. Die Wirkstoffe der erfindungsgemäßen Kombinationen oder auch die vorgemischten Bestandteile der erfindungsgemäßen Kombinationen können im Mischvorgang zugegeben werden.

Der pH-Wert der Zubereitungen kann in bekannter Weise durch Zugabe von Säuren oder Basen eingestellt werden, vorzugsweise durch Zugabe von Puffergemischen, z.B. auf Basis von Citronensäure/Citrat oder Phosphorsäure Phosphat-Puffergemischen. Vorzugsweise liegt der pH-Wert unter 10, z.B. im Bereich von 3 bis 9.

Alle Mengenangaben, Anteile und Prozentanteile sind, soweit nicht anders angegeben, auf das Gewicht und die Gesamtmenge bzw. auf das Gesamtgewicht der Zubereitungen oder der jeweiligen Mischung bezogen.

| | Schaum festiger 1 | Schaum festiger 2 | Haarspray 1 | Haarspray 2 | Haarspray 3 | Haarspray 4 | Haarspray 5 | Schaumfestiger 3 | Schaumfestiger 4 | Schaumfestiger 5 |
|---|---|---|---|---|---|---|---|---|---|---|
| Polyquaternium-4 | 0,04 | 0,04 | 0 | 0 | 0 | 0 | 0 | 0,1 | 0 | 0,1 |
| Citronensäure | 0,07 | 0,07 | 0 | 0 | 0 | 0 | 0 | 0,07 | 0,07 | 0,07 |
| Natriumbenzoat | 0,20 | 0,20 | 0 | 0 | 0 | 0 | 0 | 0,20 | 0,20 | 0,20 |
| Panthenol | 0,08 | 0,08 | 0,12 | 0,12 | 0,12 | 0,12 | 0,1 | 0 | 0,2 | 0,05 |
| Cetrimoniumchlorid | 0,15 | 0,15 | 0 | 0 | 0 | 0 | 0 | 0,3 | 0,2 | 0,15 |
| Hydroxyethyl Cetyldimonium Phosphat | 0,11 | 0,11 | 0 | 0 | 0 | 0 | 0 | 0 | 0,05 | 0,2 |
| Polyquaternium-68 | 0,70 | 0,70 | 0 | 0 | 0 | 0 | 0 | 0 | 0,6 | 0,6 |
| Niacinamid | 0,02 | 0,02 | 0,03 | 0,03 | 0,03 | 0,03 | 0,03 | 0,1 | 0 | 0,1 |
| VP/VA Copolymer | 3,35 | 3,35 | 0 | 0 | 0 | 0 | 0 | 2 | 1 | 0 |
| PEG-40 Hydrogeniertes Rhizinusöl | 0,3 | 0,3 | 0 | 0 | 0 | 0 | 0 | 0,3 | 0,3 | 0,3 |
| Octanohydroxamsäure | 0,25 | 0 | 0,25 | 0 | 0,5 | 0,25 | 0,25 | 0,1 | 0,3 | 0,25 |
| Aminomethyl Propanol | 0 | 0 | 0,87 | 0,87 | 0,87 | 0,87 | 0,87 | 0 | 0 | 0 |
| PEG/PPG-17/18 Dimethicone | 0 | 0 | 0,25 | 0,25 | 0,25 | 0,25 | 0,25 | 0 | 0 | 0 |
| Octylacrylamide/Acrylates/ Butylaminoethyl Methacrylate Copolymer | 0 | 0 | 4,93 | 4,93 | 0 | 4,93 | 0 | 0 | 0 | 0 |
| PEG-12 Dimethicone | 0 | 0 | 0,09 | 0,09 | 0,09 | 0,3 | 0,1 | 0 | 0 | 0 |
| PVP | 0 | 0 | 0 | 0 | 5 | 0 | 0 | 0 | 0 | 1 |
| Acrylates/t-Butylacrylamid Copolymer | 0 | 0 | 0 | 0 | 0 | 0 | 5 | 0 | 0 | 0 |
| Polyquaternium-11 | 0 | 0 | 0 | 0 | 0 | 0 | 0 | 0,5 | 0 | 0 |
| Polyquaternium-16 | 0 | 0 | 0 | 0 | 0 | 0 | 0 | 2 | 0 | 0 |
| Sorbitol | 0 | 0 | 0 | 0 | 0 | 0 | 0 | 0 | 2 | 0,2 |
| Amodimethicon | 0 | 0 | 0 | 0 | 0 | 0 | 0 | 0,2 | 0 | 0 |
| Behenoxydimethicon | 0 | 0 | 0 | 0 | 0 | 0 | 0 | 0 | 0,4 | 0 |
| Parfum | qs | qs | qs | qs | qs | qs | qs | qs | qs | qs |
| Alkohol denat. | 0 | 0 | Ad 100 | Ad 100 | Ad 100 | Ad 100 | Ad 100 | 2 | 0 | 0 |
| Wasser | Ad 100 | Ad 100 | 0 | 0 | 0 | 15 | 0 | Ad 100 | Ad 100 | Ad 100 |

Die Haarspray- und Schaumfestigerrezepturen beziehen sich jeweils auf die Wirklösung.

Die Haarsprays 1,2 und 5 wurden in einem Verhältnis 65% Wirklösung zu 35% Treibmittel Propan/Butan/Isobutan hergestellt. Die Haarsprays 3 und 4 wurden in einem Verhältnis 50% Wirklösung zu 50% Treibmittel Dimethylether hergestellt.
Druckstufe: 2,7 bar (bez. auf Propan/ Butan/ Isobutan)
Druckstufe: 5 bar (bez. auf Dimethylether)

Alle Schaumfestigerrezepturen wurden in einem Verhältnis 91% Wirklösung und 9% Treibmittel Proban/ Butan/ Isobutan hergestellt.
Druckstufe: 3,0 bar

### Herstellanweisung der Wirklösungen

### Herstellanweisung Haarsprays (bezogen auf Haarspray 1 bzw. 2)

Ethanol und Aminomethyl Propanol zusammengeben. Octylacrylamide/Acrylates/ Butylaminoethyl Methacrylate Copolymer langsam unter Rühren einstreuen. Octanohydroxamsäure gegebenenfalls dazugeben.

Alle restlichen Rezepturbestandteile hinzugeben; mit Parfüm abschließen.

### Herstellanweisung Schaumfestiger (bezogen auf Schaumfestiger 1 bzw. 2)

Polyquaternium-4 unter rühren in Wasser lösen und anschließend alle weiteren Rezepturbestandteile abgesehen von Parfüm, PEG-40 Hydrogenated Castor Oil und Octanohydroxamic Acid hinzugeben. Octanohydroxamic Acid gegebenenfalls unter rühren und leichtem Erwärmen separat in Wasser lösen und anschließend zum Ansatz geben. Parfum und PEG-40 Hydrogenated Castor Oil unter Rühren bei ca. 40°C aufschmelzen, zu einer klaren Lösung verarbeiten und zum Ansatz geben.

### Durchführung

### Durchführung der Evaluierung Schaumfestiger (Curl Retention)

Die Haarsträhnen (ungeschädigt, 10g) werden 15 Minuten in Wasser eingeweicht. Anschließend werden die nassen Haarsträhnen durch Trocknen mit einem Handtuch auf 15 g gebracht und die Strähnen werden gekämmt. Desweiteren wird 1 g Schaumfestiger gleichmäßig auf die Strähne aufgebracht und verteilt. Dann werden die Strähnen auf Lockenwickler aufgewickelt und verbleiben 24 Stunden bei Raumtemperatur auf den Wicklern. Nach dem Entfernen der Lockenwickler wird die curl retention optisch beurteilt. und Länge der Haarsträhnen wird prozentual miteinander verglichen. Jede Schaumfestigerformulierung wird an 3 Haartressen getestet und der prozentuale Vergleich erfolgt aus den Mittelwerten. (siehe Verbesserung aus mail vom 2.12.)

### Durchführung Performanceevaluierung Haarsprays

2 g Haarspray wird im Abstand von 30 cm gleichmäßig auf ungeschädigte 10 g schwere Haare aufgesprüht. Anschließend wird der Halt und die Festigung der Haarsträhnen von 3 geschulten Panelisten an der Strähne beurteilt.

### Ergebnisse

Der Schaumfestiger mit 0,25% Octanohydroxamsäure (Schaumfestiger 1) eine bessere Curl Retention als der Schaumfestiger ohne den Inhaltsstoff (Schaumfestiger 2).

Verglichen mit Schaumfestiger 2 (ohne Octanohydroxamsäure) zeigt der Schaumfestiger 1 (mit 0,25% Octanohydroxamic Acid) eine um 17,25% verringerte Haarlänge und damit eine verbesserte Curl Retention Leistung.

### Ergebnisse Haarspray

**Tabelle 1: Ergebnis Haarspray 1 (0,25% Octanohydroxamsäure)**

| | Panelist 1 | Panelist 2 | Panelist 3 | Mittelwert |
|---|---|---|---|---|
| Halt | 4 | 5 | 5 | 4,7 |
| Festigung | 5 | 5 | 4 | 4,7 |

**Tabelle 2: Ergebnis Haarspray 2 (Formulierung ohne Octanohydroxamsäure)**

| | Panelist 1 | Panelist 2 | Panelist 3 | Mittelwert |
|---|---|---|---|---|
| Halt | 3 | 3 | 4 | 3,3 |
| Festigung | 3 | 4 | 4 | 3,7 |

### Erklärung der Bewertung:

1 - sehr geringer Halt bzw. Festigung
5 - sehr hoher Halt bzw. Festigung

Haarspray 1 (mit 0,25% Octanohydroxamsäure) und Haarspray 2 (gleiche Formulierung ohne Octanohydroxamsäure) wurden auf Strähnen im Labor von 3 geschulten Panelisten im Bezug auf Halt und Festigung bewertet.

Die Formel mit Octanohydroxamsäure schnitt in beiden Parametern besser ab.

## Patentansprüche

1. Haarkosmetische Zubereitungen mit einem Gehalt 0,01 bis 10 Gew.-%, insbesondere 0,05 bis 3,0 Gew.-%, an physiologisch verträglicher Octanohydroxamsäure, bezogen auf das Gesamtgewicht der Zusammensetzung und
- einem oder mehreren kationischen Polymeren, welches oder welche gewählt werden aus der Gruppe Vinylpyrrolidon/Vinylimidazolium-methochlorid-Copolymere, quaternisierte Vinylpyrrolidon/Dialkylaminoalkylmethacrylat-Copolymere, kationische Cellulose-Derivate, wie z.B. Hydroxyethylcellulose/Dimethyl-alkylammoniumchlorid-Copolymere sowie Terpolymere aus Vinylcaprolactam/-Vinylpyrrolidon mit Dimethylaminoethylmethacrylat bzw. Vinylimmidazoliniummetho-chlorid und Acrylamidocopolymere, Hydroxyethyl Cellulose Dimethyl Diallylammonium Chloride Copolymer (Polyquaternium-4), Vinylpyrrolidon/ Vinylimidazol/Methacrylamid/-quaternisiertes Vinylimidazol (Polyquaternium-68), Vinylpyrrolidone/ quaternisiertes Dimethylaminoethylmethacrylate Copolymer (Polyquaternium-11), vorzugsweise Polyquaternium-4, Polyquaternium-11 und Polyquaternium-68 und
- einem oder mehreren nichtionischen Polymeren, welches oder welche gewählt werden aus der Gruppe Homopolymere des Vinylpyrrolidons, Homopolymere des N-Vinylformamids, Copolymerisate aus Vinylpyrrolidon und Vinylacetat, Terpolymere aus Vinylpyrrolidon, Vinylacetat und Propionat, Polyvinylcaprolactam, Polyvinylamide und deren Salze sowie Copolymere aus Vinylpyrrolidon und Dimethylaminoethylmethacrylat und Terpolymere aus Vinylcaprolactam, Vinylpyrrolidon und Dimethylaminomethacrylat, vorzugsweise Copolymerisate aus Vinylpyrrolidon und Vinylacetat sowie Homopolymerisate des Vinylpyrrolidons.

2. Haarkosmetische Zubereitungen mit einem Gehalt 0,01 bis 10 Gew.-%, insbesondere 0,05 bis 3,0 Gew.-%, an physiologisch verträglicher Octanohydroxamsäure und
- einem oder mehreren nichtionischen Polymeren, welches oder welche gewählt werden aus der Gruppe Homopolymere des Vinylpyrrolidons, Homopolymere des N-Vinylformamids, Copolymierisate aus Vinylpyrrolidon und Vinylacetat, Terpolymere aus Vinylpyrrolidon, Vinylacetat und Propionat, Polyvinylcaprolactam, Polyvinylamide und deren Salze sowie Copolymere aus Vinylpyrrolidon und Dimethylaminoethylmethacrylat und Terpolymere aus Vinylcaprolactam, Vinylpyrrolidon und Dimethylaminomethacrylat, vorzugsweise Copolymerisate aus Vinylpyrrolidon und Vinylacetat sowie Homopolymerisate des Vinylpyrrolidons sowie
- einem oder mehreren amphoteren Polymeren, welches oder welche gewählt werden aus der Gruppe Copolymerisate aus N-Octylacrylamid, (Meth)Acrylsäure und tert.-Butylaminoethylmethacrylat, Copolymerisate aus Methacryloylethylbetain und Alkylmethacrylaten, Copolymerisate aus Carboxylgruppen oder Sulfongruppen enthaltenden Monomeren, z.B. (Meth)Acrylsäure und Itaconsäure, mit basischen, insbesondere Aminogruppen enthaltenden Monomeren wie z.B. Mono- bzw. Dialkylaminoalkyl(meth)acrylaten und/oder Mono- bzw. Dialkylaminoalkyl(meth)acrylamiden, Copolymere aus N-Octylacrylamid, Methyl-methacrylat, Hydroxypropylmethacrylat, N-tert.-Butylaminoethylmethacrylat und Acrylsäure, vorzugsweise Octylacrylamide/Acrylates/ Butylaminoethyl Methacrylate Copolymer.

3. Haarkosmetische Zubereitungen nach Anspruch 2 mit einem Gehalt 0,01 bis 10 Gew.-%, insbesondere 0,05 bis 3,0 Gew.-%, an physiologisch verträglicher Octanohydroxamsäure und einem oder mehreren Treibmitteln, **dadurch gekennzeichnet, dass** das oder die Treibmittel bevorzugt gewählt werden aus der Gruppe der Kohlenwasserstoffe wie Butan, Isobutan und Propan oder Dimethylether.

## Claims

1. Cosmetic hair preparations having a content of 0.01 to 10 wt%, especially 0.05 to 3.0 wt% of physiologically acceptable octanohydroxamic acid, based on the total weight of the composition, and
- one or more cationic polymers selected from the group of vinylpyrrolidone/vinylimidazolinium methochloride copolymers, quaternized vinylpyrrolidone/dialkylaminoalkyl methacrylate copolymers, cationic cellulose derivatives, such as, for example, hydroxyethyl cellulose/dimethyl alkylammonium chloride copolymers and also terpolymers of vinylcaprolactam/vinylpyrrolidone with dimethylaminoethyl methacrylate or vinylimidazolinium methochloride and acrylamido copolymers, Hydroxyethyl Cellulose Dimethyl Diallylammonium Chloride Copolymer (Polyquaternium-4), vinylpyrrolidone/vinylimidazole/methacrylamide/quaternized vinylimidazole (Polyquaternium-68), vinylpyrrolidone/quaternized dimethylaminoethyl methacrylate copolymer (Polyquaternium-11), preferably Polyquaternium-4, Polyquaternium-11 and Polyquaternium-68 and
- one or more non-ionic polymers selected from the group of homopolymers of vinylpyrrolidone, homopolymers of N-vinylformamide, copolymers of vinylpyrrolidone and vinyl acetate, terpolymers of vinylpyrrolidone, vinyl acetate and propionate, polyvinylcaprolactam, polyvinylamides and the salts thereof and also copolymers of vinylpyrrolidone and dimethylaminoethyl methacrylate and terpolymers of vinylcaprolactam, vinylpyrrolidone and dimethylamino methacrylate, preferably copolymers of vinylpyrrolidone and vinyl acetate and also homopolymers of vinylpyrrolidone.

2. Cosmetic hair preparations having a content of 0.01 to 10 wt%, especially 0.05 to 3.0 wt% of physiologically acceptable octanohydroxamic acid, and
- one or more non-ionic polymers selected from the group of homopolymers of vinylpyrrolidone, homopolymers of N-vinylformamide, copolymers of vinylpyrrolidone and vinyl acetate, terpolymers of vinylpyrrolidone, vinyl acetate and propionate, polyvinylcaprolactam, polyvinylamides and the salts thereof and also copolymers of vinylpyrrolidone and dimethylaminoethyl methacrylate and terpolymers of vinylcaprolactam, vinylpyrrolidone and dimethylamino methacrylate, preferably copolymers of vinylpyrrolidone and vinyl acetate and also homopolymers of vinylpyrrolidone, and also
- one or more amphoteric polymers selected from the group of copolymers of N-octylacrylamide, (meth)acrylic acid and tert-butylaminoethyl methacrylate, copolymers of methacryloyl ethyl betaine and alkyl methacrylates, copolymers of monomers comprising carboxyl groups or sulfone groups, e.g. (meth)acrylic acid and itaconic acid, having basic monomers, especially monomers comprising amino groups, such as, for example, mono- or dialkylaminoalkyl (meth)acrylates and/or mono- or dialkylaminoalkyl (meth)acrylamides, copolymers of N-octylacrylamide, methyl methacrylate, hydroxypropyl methacrylate, N-tert-butylaminoethyl methacrylate and acrylic acid, preferably Octylacrylamide/Acrylates/Butylaminoethyl Methacrylate Copolymer.

3. Cosmetic hair preparations according to Claim 2, having a content of 0.01 to 10 wt%, especially 0.05 to 3.0 wt% of physiologically acceptable octanohydroxamic acid and one or more propellants, **characterized in that** the propellant(s) are preferably selected from the group of hydrocarbons such as butane, isobutane and propane or dimethyl ether.

## Revendications

1. Préparations cosmétiques capillaires ayant une teneur de 0,01 à 10 % en poids, notamment de 0,05 à 3,0 % en poids, en acide octanohydroxamique physiologiquement compatible, par rapport au poids total de la composition, et en
- un ou plusieurs polymères cationiques, qui sont choisis dans le groupe constitué par les copolymères de vinylpyrrolidone/méthochlorure de vinylimidazolium, les copolymères de vinylpyrrolidone quaternisée/méthacrylate de dialkylaminoalkyle, les dérivés de cellulose cationiques, tels que p. ex. les copolymères d'hydroxyéthylcellulose/chlorure de diméthylalkylammonium, ainsi que les terpolymères de vinylcaprolactame/vinylpyrrolidone avec du méthacrylate de diméthylaminoéthyle ou du méthochlorure de vinylimidazolinium, et les copolymères d'acrylamido, le copolymère de chlorure d'hydroxyéthylcellulose-diméthyldiallylammonium (polyquaternium-4), le vinylpyrrolidone/vinylimidazole/méthacrylamide/vinylimidazole quaternisé (polyquaternium-68), le copolymère de vinylpyrrolidone/méthacrylate de diméthylaminoéthyle quaternisé (polyquaternium-11), de préférence polyquaternium-4, polyquaternium-11 et polyquaternium-68, et en
- un ou plusieurs polymères non ioniques, qui sont choisis dans le groupe constitué par les homopolymères de vinylpyrrolidone, les homopolymères de N-vinylformamide, les copolymères de vinylpyrrolidone et d'acétate de vinyle, les terpolymères de vinylpyrrolidone, d'acétate de vinyle et de propionate, le polyvinylcaprolactame, le polyvinylamide et ses sels, ainsi que les copolymères de vinylpyrrolidone et de méthacrylate de diméthylaminoéthyle, et les terpolymères de vinylcaprolactame, de vinylpyrrolidone et d'aminométhacrylate de diméthyle, de préférence les copolymères de vinylpyrrolidone et d'acétate de vinyle, ainsi que les homopolymères de vinylpyrrolidone.

2. Préparations cosmétiques capillaires ayant une teneur de 0,01 à 10 % en poids, notamment de 0,05 à 3,0 % en poids, en acide octanohydroxamique physiologiquement compatible, et en
- un ou plusieurs polymères non ioniques, qui sont choisis dans le groupe constitué par les homopolymères de vinylpyrrolidone, les homopolymères de N-vinylformamide, les copolymères de vinylpyrrolidone et d'acétate de vinyle, les terpolymères de vinylpyrrolidone, d'acétate de vinyle et de propionate, le polyvinylcaprolactame, le polyvinylamide et ses sels, ainsi que les copolymères de vinylpyrrolidone et de méthacrylate de diméthylaminoéthyle, et les terpolymères de vinylcaprolactame, de vinylpyrrolidone et d'aminométhacrylate de diméthyle, de préférence les copolymères de vinylpyrrolidone et d'acétate de vinyle, ainsi que les homopolymères de vinylpyrrolidone, ainsi qu'en
- un ou plusieurs polymères amphotères, qui sont choisis dans le groupe constitué par les copolymères de N-octylacrylamide, d'acide (méth)acrylique et de méthacrylate de tert.-butylaminoéthyle, les copolymères de méthacryloyléthylbétaïne et de méthacrylates d'alkyle, les copolymères de monomères contenant des groupes carboxyle ou des groupes sulfone, p. ex. l'acide (méth)acrylique et l'acide itaconique, avec des monomères basiques, notamment contenant des groupes amino, tels que p. ex. les (méth)acrylates de mono- ou dialkylaminoalkyle et/ou les mono- ou dialkylaminoalkyl(méth)acrylamides, les copolymères de N-octylacrylamide, de méthacrylate de méthyle, de méthacrylate d'hydroxypropyle, de méthacrylate de N-tert.-butylaminoéthyle et d'acide acrylique, de préférence le copolymère d'octylacrylamide/acrylates/méthacrylate de butylaminoéthyle.

3. Préparations cosmétiques capillaires selon la revendication 2, ayant une teneur de 0,01 à 10 % en poids, notamment de 0,05 à 3,0 % en poids, en acide octanohydroxamique physiologiquement compatible, et en un ou plusieurs agents gonflants, **caractérisées en ce que** le ou les agents gonflants sont de préférence choisis dans le groupe constitué par les hydrocarbures tels que le butane, l'isobutane et le propane ou l'éther diméthylique.
